# EUROPÄISCHE PATENTSCHRIFT

(11) **EP 2 148 924 B1**
(45) Veröffentlichungstag und Bekanntmachung des Hinweises auf die Patenterteilung: **25.06.2014**
(21) Anmeldenummer: 08784498.1
(22) Anmeldetag: 21.05.2008
(51) Int. Cl.: C12N 9/80, C12P 19/28, C12N 5/10

(54) **NUKLEINSÄUREMOLEKÜLE CODIEREND FÜR EIN PROTEIN MIT DEACETYLASE-AKTIVITÄT, BESAGTES PROTEIN, SOWIE VERFAHREN ZUR HERSTELLUNG VON CHITOSAN**
NUCLEIC ACID MOLECULES CODING FOR A PROTEIN WITH DEACETYLASE ACTIVITY, SAID PROTEIN, AND METHOD FOR THE PRODUCTION OF CHITOSAN
MOLÉCULES D'ACIDE NUCLÉIQUE CODANT UNE PROTÉINE À ACTIVITÉ DÉSACÉTYLASE, LADITE PROTÉINE, ET PROCÉDÉS DE PRODUCTION DE CHITOSANE

(30) Priorität: 22.05.2007 DE 102007024085
(43) Veröffentlichungstag der Anmeldung: 03.02.2010
(73) Patentinhaber: Westfälische Wilhelms-Universität Münster, 48149 Münster (DE)
(72) Erfinder: KINGA JASZCZUK, Beate, 58167 Münster (DE); MOERSCHBACHER, Bruno, 48161 Münster (DE); SCHAAF, Andreas, 48145 Münster (DE)
(74) Vertreter: Michalski Hüttermann & Partner Patentanwälte
(86) Internationale Anmeldenummer: PCT/EP2008/004079
(87) Internationale Veröffentlichungsnummer: WO 2008/141818

(56) Entgegenhaltungen:
- EP-A- 1 884 567
- DE-A1- 19 810 349
- DATABASE EMBL [Online] 20. April 2007 (2007-04-20), "Ps68 Wheat stripe rust fungus cDNA library Puccinia striiformis f. sp. tritici cDNA 5' similar to carbohydrate deacetylase, mRNA sequence." XP002495732 gefunden im EBI accession no. EMBL:ES321999 Database accession no. ES321999
- RINAUDO ET AL: "Chitin and chitosan: Properties and applications" PROGRESS IN POLYMER SCIENCE, PERGAMON PRESS, OXFORD, GB, Bd. 31, Nr. 7, 1. Juli 2006 (2006-07-01), Seiten 603-632, XP005623018 ISSN: 0079-6700
- HIRANO S: "Chitin and Chitosan" 2005, WILEY INTERSCIENCE (ULLMANN'S ENCYCLOPEDIA OF INDUSTRIAL CHEMISTRY ) , WILEY-VCH VERLAG GMBH & CO. KGAA, WEINHEIM , XP002495731 das ganze Dokument, insbesondere Absatz "Uses" (S.7-9)
- TSIGOS I ET AL: "Chitin deacetylases: new, versatile tools in biotechnology" TRENDS IN BIOTECHNOLOGY, ELSEVIER PUBLICATIONS, CAMBRIDGE, GB, Bd. 18, Nr. 7, 1. Juli 2000 (2000-07-01), Seiten 305-312, XP004908536 ISSN: 0167-7799
- EL GUEDDARI ET AL: "Chitosans in plant protection" SOUTH AFRICAN JOURNAL OF BOTANY - SUID-AFRIKAANS TYDSKRIFT VIRPLANTKUNDE, FOUNDATION FOR EDUCATION, SCIENCE AND TECHNOLOGY, PRETORIA, SA, Bd. 73, Nr. 2, 1. April 2007 (2007-04-01), Seite 286, XP022017916 ISSN: 0254-6299
- BLAIR DAVID E ET AL: "Structure and mechanism of chitin deacetylase from the fungal pathogen Colletotrichum lindemuthianum" BIOCHEMISTRY, Bd. 45, Nr. 31, August 2006 (2006-08), Seiten 9416-9426, XP002495730 ISSN: 0006-2960 in der Anmeldung erwähnt
- TANAKA TAKESHI ET AL: "Concerted action of diacetylchitobiose deacetylase and exo-beta-D-glucosaminidase in a novel chitinolytic pathway in the hyperthermophilic archaeon Thermococcus kodakaraensis KOD1" JOURNAL OF BIOLOGICAL CHEMISTRY, AMERICAN SOCIETY OF BIOLOCHEMICAL BIOLOGISTS, BIRMINGHAM,; US, Bd. 279, Nr. 29, 16. Juli 2004 (2004-07-16), Seiten 30021-30027, XP002415753 ISSN: 0021-9258 in der Anmeldung erwähnt

## Beschreibung

### Gebiet der Erfindung

Die vorliegende Erfindung betrifft Nukleinsäuremoleküle gemäß dem Oberbegriff des Anspruchs 1 codierend für ein Protein mit Deacetylase-Aktivität, besagtes Protein sowie Verfahren zur Herstellung derselben.

### Hintergrund der Erfindung

Besagtes Protein mit Deacetylase-Aktivität ist insbesondere interessant für die Herstellung von Chitosan, das aufgrund seiner besonderen Eigenschaften in jüngerer Zeit erhöhte Aufmerksamkeit gefunden hat.

Bei Chitosan handelt es sich um den Oberbegriff für lineare Copolymere aus ß-1,4-glycosidisch verknüpften N-Acetyl-Glucosamin- (GlcNAc) und Glucosamin-Resten(GlcN).

Im Prinzip handelt es sich dabei um partiell deacetyliertes Chitin (Poly-GlcNAc); es gibt aus diesem Grund keinen genau definierten Übergang zwischen Chitin und Chitosan. In der Regel spricht man ab einem Deacetylierungsgrad (d.h. ab einem GlcN-Anteil) von mehr als 40 % von Chitosan. Aufgrund der Deacetylierung ist Chitosan im Unterschied zu Chitin in organischen Säuren löslich.

Man unterscheidet weiterhin zwischen niedermolekularen (M ∼ 150 000 D), mittelmolekularen (M ∼ 400 000 D) und hochmolekularen (M - 600 000 D) Chitosanen.

Chitosan ist eines der wenigen Biopolymere, das unter physiologischen Bedingungen eine positive Nettoladung trägt. Aufgrund dieser Tatsache interagiert Chitosan mit anionischen Substanzen wie Proteinen, DNA, Fettsäuren, und Phospholipiden. Diese physiko-chemischen Interaktionen werden für die verschiedenen biologischen Wirkungen von Chitosan verantwortlich gemacht.

Chitosan kommt in der Natur nur sehr selten vor; es findet sich hauptsächlich in der Zellwand von Pilzen der Klasse der Zygomycetes.

Chitosan für kommerzielle Zwecke wird daher aus Chitin hergestellt. Chitin ist ein weit verbreitetes Biopolymer und kommt insbesondere in den Zellwänden von Pilzen sowie im Exoskelett der meisten Arthropoden, insbesondere Crustaceen, vor. Vor allem die in der Aquakultur sowie dem Krabbenfang in großer Menge anfallenden Crustaceenschalen stellen eine kostengünstige Quelle für Chitin dar.

Im Stand der Technik wird Chitosan auf chemischem Weg aus Chitin hergestellt. Dazu werden die Crustaceenschalen zunächst vermahlen und anschließend zur Entfernung von Kalk, das einen Gewichtsanteil von 30 % (w/w) an dem Schalenmaterial stellt, mit einem Überschuß von HCl (0.25-1 %, 12-24 h, Raumtemperatur) behandelt. Anschließend wird gewaschen, und dann folgt ein Deproteinierungsschritt unter Verwendung eines Überschusses an heißer, wässriger NaOH (0.25-1 M, 1-3 h, 60-70°C). Das Produkt wird dann gewaschen, neutralisiert und getrocknet.

Das auf diese Weise gewonnene Chitin wird dann einer partiellen Deacetylierung unterzogen. Hierzu wird das Chitin wiederholt mit einer heißen konzentrierten Lauge, bevorzugt NaOH, behandelt (35-50 %, 1-3 h, 90-130 °C).

Es schließt sich in einigen Fällen ein Entfärbungsschritt, z.B. mit UV, O₃ oder H₂O₂, an. Das Produkt wird dann gewaschen, neutralisiert und getrocknet.

Das beschriebene Verfahren führt zu Produkten mit einem zufälligen Acetylierungsmuster, und überdies zu einer teilweisen Depolymerisierung des Polymers.

Überdies kann das Produkt unlösliche Bereiche mit sehr hohem Acetylierungsgrad aufweisen, die aus den inneren, kristallinen Bereichen der Chitin-Partikel herrühren.

Das so hergestellte Chitosan weist daher in Bezug auf Polymerisierungsgrad, Acetylierungsgrad und Acetylierungsmuster eine große Heterogenität auf. Gleichwohl ist dieses Produkt für eine Reihe von Anwendungen geeignet.

Anspruchsvollere Anwendungen erfordern jedoch Chitosane höherer Qualität, d.h. Chitosane mit genauer definiertem Polymerisierungsgrad (DP), Acetylierungsgrad (DA) und Acetylierungsmuster.

Solche Chitosane lassen sich, wenn überhaupt, nur unter großem Aufwand aus den wie oben beschriebenen Produkten herstellen bzw. aufreinigen.

Seit einiger Zeit wird daher der Ansatz verfolgt, Chitosan mit Hilfe geeigneter Chitin-Deacetylasen aus Chitin herzustellen. Es wird vermutet, dass insbesondere marine Mikroorganismen Chitin-Deacetylasen aufweisen, um z.B. Exoskelette von Crustaceen-Kadavern abbauen zu können.

Tanaka et al. (J Biol Chem. 2004; 279: 30021-7) haben eine Diacetylchitobiose-Deacetylase aus dem hyperthermophilen Archaebacterium *Thermococcus kodakaraensi* beschrieben. Die rekombinante Deacetylase zeigte Deacetylase-Aktivität gegenüber N-Acetylchitooligosacchariden; die deacetylierende Domäne weist eine Spezifität für den nichtreduzierenden GlcNAc-Rest auf. Das Enzym wies ebenfalls eine Deacetylase-Aktivität gegenüber GlcNAc-Monomeren auf. Die abgeleitete Aminosäuresequenz wurde der LmbE-Proteinfamilie zugeordnet, in welche auch N-Acetylglucosaminyl-phosphatidylinositol-De-N-acetylasen und 1-D-myo-inosityl-2-acetamido-2-deoxy-alpha-D-glucopyranoside-Deacetylasen eingeordnet werden.

Es stellte sich heraus, dass sich die *Thermococcus*-Deacetylase nicht für die wirtschaftlich sinnvolle Produktion von Chitosan aus Chitin eignet. Es lässt sich allenfalls für die Produktion weniger, spezifischer Oligomere verwenden.

Hinzu kommt, dass die Expressionsrate dieses Proteins verhältnismäßig schlecht ist, so dass das gesuchte Protein nur in geringen Mengen hergestellt werden kann. Überdies weist das Protein sein Aktivitätsmaximum bei 75° C und pH 8.5 auf. Diese Bedingungen sind für viele Anwendungen nicht geeignet. Hinzu kommt, dass besagtes Protein nur die erste GlcNac-Position am nichtreduzierenden Ende deacetyliert und maximal ein Pentamer als Substrat akzeptiert, beim Hexamer oder gar hochpolymeren Chitosanen jedoch keine Deacetylase-Aktivität mehr auftritt.

In der Summe ist die einzig denkbare kommerzielle Anwendung daher die Deacetylierung der ersten, nichtreduzierenden Position von Oligomeren mit einer Maximallänge von 5 Einheiten.

Blair et al. (Biochemistry, 2006, 45 (31), 9416 -9426) haben erstmals die Struktur und den Wirkmechanismus einer Chitin-Deacetylase des pathogenen Pilzes Colletotrichum lindemuthianum (Erreger der Brennfleckenkrankheit) aufgezeigt. Auch diese Deacetylase weist eine Reihe von Nachteilen auf, die sie für die industrielle Eignung nicht oder zumindest kaum geeignet machen. So kann diese Deacetylase nur maximal jede zweite Zuckerposition deacetylieren. Daher liegt der theoretisch erreichbare maximale Deacetylierungsgrad bei 50%. Hinzu kommt, dass mit dieser Deacetylase eine blockweise Deacetylierung nicht möglich ist.

### Beschreibung der Erfindung

Es ist daher eine Aufgabe der vorliegenden Erfindung, ein Deacetylase-Enzym mit Deacetylase-Aktivität gegenüber GlcNAc-Molekülen und -Polymeren (Chitin, Chitosanen) bereitzustellen, das sich in großen Mengen kostengünstig herstellen läßt und Aktivitätsbedingungen aufweist, die es besser für industrielle Prozesse geeignet machen.

Eine andere Aufgabe der vorliegenden Erfindung liegt darin, ein Verfahren zur Herstellung von Chitosan aus Chitin bereitzustellen, das wirtschaftlicher und umweltfreundlicher ist als das eingangs beschrieben Verfahren und/oder die Herstellung von genauer definierten Chitosanen ermöglicht.

Es ist eine weitere Aufgabe der vorliegenden Erfindung, die Aminosäuresequenz sowie die betreffende Nukleinsäuresequenz für besagtes Deacetylase-Enzym bereitzustellen.

Diese Aufgabe wird mit einem Nukleinsäuremolekül, einem Protein bzw. einem Verfahren gemäß den unabhängigen Ansprüchen gelöst. Die Unteransprüche geben bevorzugte Ausführungsformen an.

Weitere Chitin-Deacylasen sind u.a. aus der EMBL-Datenbank, Stichwort "Ps68 Wheat stripe rust fungus cDNA library Puccinia striiformis f. sp. tritici cDNA 5' similar to carbohydrate deacetylase, mRNA sequence" bekannt.

Demnach ist ein Nukleinsäuremolekül vorgesehen, das ausgewählt ist aus der Gruppe bestehend aus:
a) Nukleinsäuremoleküle, die für eine Form des Polypeptids mit der abgeleiteten Aminosäuresequenz gemäß SEQ ID No: 3 codieren, wobei besagtes Polypeptid eine Deacetylase-Aktivität aufweist;
b) Nukleinsäuremoleküle, die die Nukleotidsequenz gemäß SEQ ID No: 1 oder SEQ ID No: 2 aufweisen, und die für eine Form des besagten Polypeptids codieren;
c) Nukleinsäuremoleküle, die für ein Fragment oder ein Derivat eines Polypeptids, das durch ein Nukleinsäuremolekül gemäß a) oder b) codiert wird, codieren, wobei in besagtem Derivat ein oder mehrere Aminosäurereste im Vergleich zu besagtem Polypeptid konservativ substituiert sind, und wobei besagtes Fragment oder Derivat eine Deacetylase-Aktivität hat, und die eine mindestens 95 %ige Sequenzidentität mit einem Nukleinsäuremolekül gemäß a) oder b) aufweisen,
d) oder der komplementäre Strang eines Nukleinsäuremoleküls gemäß a)- e).

Unter dem Begriff "Fragment" soll im Folgenden ein Polypeptid verstanden werden, das mindestens eine Aminosäure weniger als das ursprünglich beanspruchte Polypeptid aufweist.

Unter dem Begriff "Derivat" soll im Folgenden ein Polypeptid verstanden werden, das mindestens eine Aminosäuren-Substitution im Vergleich zu dem ursprünglich beanspruchten Polypeptid aufweist.

Grundsätzlich sind jedoch durch die obige Definition auch solche Nukleinsäuremoleküle erfasst, die zwar eine von den beanspruchten Sequenzen SEQ ID No: 1 oder SEQ ID No: 2 abweichende Sequenz aufweisen, bei denen sich diese Sequenzunterscheide jedoch nicht in Sequenzabweichungen des codierten Polypeptids äußern, da es sich um sogenannte "stille Punktmutationen" (Punktmutationen in der dritten Stelle eines Tripletts) handelt, die aufgrund der Degenerierung des Genetischen Codes nicht zu einem Aminosäureaustausch führen (sogenannte "wobble"-Mutationen).

Unter dem Begriff "konservativ substituiert" soll im folgenden eine Substitution eines Nukleinsäuremoleküls, d.h. eine oder mehrere Basenaustauschmutationen, verstanden werden, die dazu führen, dass eine an einer Stelle ursprünglich vorhandene Aminosäure durch eine Aminosäure ersetzt wird, die die Eigenschaften des Polypeptids nicht oder nicht wesentlich verändert. In der Regel trifft dies für substituierende Aminosäuren mit ähnlichen chemischen Eigenschaften zu.

So fasst man u.A. die Aminosäuren Alanin, Glycin, Isoleucin, Leucin, Methionin und Valin zu den aliphatischen Aminosäuren zusammen. Phenylalanin, Tryptophan und Tyrosin werden zu den aromatischen Aminosäuren gezählt, Serin, Threonin, Asparagin, Glutamin und Tyrosin zählen zu den polaren Aminosäuren, Lysin, Arginin und Histidin zählen zu den basischen Aminosäuren, Asparaginsäure (Aspartat) und Glutaminsäure (Glutamat) zählen zu den sauren Aminosäuren und Cystein und Tyrosin zu den Aminosäuren mit ionisierbaren Resten.

Bei dem codierten Polypeptid mit Deacetylase-Aktivität handelt es sich, wie die Erfinder überraschend gefunden haben, um eine Chitin-Deacetylase (CDA), und zwar um eine Chitin-Deacetylase aus Weizenschwarzrost *(Puccinia graminis).*

Hinweise auf die Existenz einer Chitin-Deacetylase ergaben sich aus Experimenten mit axenischen Kulturen des Pilzes, die weltweit einzig in den Laboren der Erfinder durchgeführt werden können. Hier konnte zum Einen nachgewiesen werden, dass *P. graminis* seine Zellwand ab dem Moment des Eindringens in die Pflanze von Chitin nach Chitosan modifiziert, zum Zweiten fanden sich in Zymogrammen mit Chitin-Substrat wenigstens zwei (Iso-)Formen Chitin-deacetylierender Enzyme. Den Erfindern gelang es daraufhin durch Durchmusterung einer Lambda-cDNA-Bank die Sequenz einer putativen Polysaccharid-Deacetylase zu isolieren. Die primäre Identifikation fußte auf der Erkennung des in der PFAM-Datenbank hinterlegten polysacch_deac_1-Musters. Mit der erlangten Information war es den Erfindern möglich, die korrespondierende genomischen Sequenz aus dem *P. graminis-*Genom zu isolieren und hinsichtlich ihrer Intron-/Exon-Struktur zu analysieren.

Hinsichtlich der Peptidsequenz weist das Protein das bereits benannte Sequenzpattern einer typischen Polysaccharid-Deacetylase auf (PFAM: polysacch_deac_1). Dieses Pattern erstreckt sich nahezu über die volle Länge der Aminosäuresequenz, besteht aber nur aus wenigen konservierten Positionen und längeren, intervenierenden, variablen Bereichen. N-terminal trägt das Protein zusätzlich ein durch Signal_P eindeutig erkanntes Sekretionssignal, weshalb die Erfinder eine extrazelluläre Lokalisation des Proteins vermuteten.

Die Erfinder haben festgestellt, dass Pilze der Art *Puccinia graminis* (wissenschaftlich *Puccinia graminis* f.sp. *tritici;* Abk.: "Pgt") im Augenblick der Infektion den in die Wirtspflanze eindringenden Teil ihrer Zellwand partiell deacetylieren. Die Erfinder vermuten, dass die Pilze sich auf diese Weise tarnen, um eine antimykotische Abwehrreaktion der Pflanze zu unterdrücken.

Die Erfinder haben daraufhin diese Deacetylase vorliegend erstmals beschrieben, isoliert, ihre Wirkung nachgewiesen und ihre Eignung zur industriellen Herstellung von Chitosan erkannt und beschrieben.

Im Speziellen vermuten die Erfinder eine herausragende Eignung derartiger Enzyme zur Produktion von Chitosanen mit nicht zufälligen und nicht blockweisen Acetylierungsmuster. Solche Chitosanprodukte könnten unter Anderem in der Medizin und im Pflanzenschutz Anwendung finden, da Ihre biologische Aktivität stark von benannten speziellen Verteilungsmustern abhängt, die auf konventionell chemischem Wege nicht darstellbar sind.

Besonders bevorzugt ist dabei ein Nukleinsäuremolekül vorgesehen, das aus einer sekretorischen Signalsequenz sowie dem besagten Nukleinsäuremolekül codierend für die genannte Deacetylase besteht.

Die erwähnte Signalsequenz codiert für ein sekretorisches Signalpeptid. Ein solches Signalpeptid ist ein Bestandteil eines Proteins, der zusammen mit der Sequenz für das eigentliche Protein auf der cDNA codiert ist, und der Informationen darüber enthält, wie und wohin dieses Protein innerhalb der Zelle transportiert werden soll. Proteine, die z.B. für den Transport in das Endoplasmatische Retikulum (ER) und damit u.U. für die spätere Sekretion bestimmt sind, besitzen in ihrer Aminosäuresequenz ein Signal, das sie spezifisch zur ER-Membran zielleitet. Bei sekretorischen Proteinen besteht das Signal aus einer 15 bis 40 Aminosäuren langen N-terminal lokalisierten Aminosäuresequenz. Obwohl die Primärstruktur dieser Signalpeptide nicht konserviert ist, können drei verschiedene Bereiche unterschieden werden: ein zentraler hydrophober Kern wird N-terminal von positiv geladenen Aminosäuren und C-terminal von polaren Aminosäuren flankiert. In den meisten Fällen wird das Signalpeptid nach dem Membrandurchtritt proteolytisch vom eigentlichen Protein durch das Enzym Signalpeptidase abgespalten. Die Schnittstelle wird dabei durch kleine Aminosäurereste in den Positionen -3 und -1 der C-terminalen polaren Region der Signalsequenz definiert.

Ein typisches eukaryontisches Signalpeptid, das den Transport des zu synthetisierenden Proteins ins ER bewerkstelligt, weist z.B. folgende Aminosäuresequenz auf:
MMSFVSLLVGIFWATEAEQLTKCEVFQ

Die für das Signalpeptid codierende Sequenz wird, da sie im Bereich des 5'-Endes der Nukleinsäuresequenz angeordnet ist, bei der Translation am Ribosom als erstes synthetisiert, so dass sich, wenn das Signalpeptid weit genug aus dem Ribosom herausragt, das SRP ("Signal Recognition Particle", ein mittelgroßes Riboprotein) an die naszierende Polypeptidkette und das Ribosom anlagert und die Translation stoppt. An der Oberfläche des (rauhen) ER befindet sich ein SRP-Rezeptor, der das Ribosom bindet und in Position an das Translocon bringt. Das SRP wird daraufhin abgespalten und kann erneut zur Markierung dienen. Die Polypeptidkette wird nun durch das Translocon in das Lumen des ER weitersynthetisiert. Anschließend entfernt das Enzym Signalpeptidase die Signalsequenz. Ist die Translation abgeschlossen, wird das Protein im ER-Lumen - ggf. mit Hilfe von Chaperonen - gefaltet. Hinzu kommt ggf. weitere Schritte, die unter dem oben bereits eingeführten Begriff der posttranslationalen Modifikation zusammengefasst werden.

In der Biotechnologie macht man sich solche Signalpeptide zunutze, um sicherzustellen, dass das herzustellende Protein in das die Zellen umgebende Medium sezerniert wird. Hierzu wird ein Expressionssystem verwendet, bei dem eine für das herzustellende Protein codierende cDNA in einen 3' gelegenen Bereich einer für ein Signalpeptid codierenden cDNA einkloniert wird. In der Regel geschieht das über eine downstream von der Signalsequenz gelegene Restrictionsschnittstelle. Da diese in der Regel mindestens zwei Nukleotidtripletts aufweist, erhält man auf diese Weise ein Konstrukt, bei welchem die Signalsequenz und die für das herzustellende Protein codierdende Sequenz durch mindestens zwei Nukleotidtripletts getrennt ist; das tranlatierte Protein weist also mindestens zwei Aminosäurereste zwischen dem Signalpeptid und dem herzustellenden Protein auf. Demnach ist ein Nukleinsäuremolekül vorgesehen, das für ein Signalpeptid codiert, und das dadurch gekennzeichnet ist, dass das Nukleinsäuremolekül im Bereich seines 3'-Endes eine Schnittstelle für eine Restriktionsendonuklease aufweist.

Häufig weisen native Nukleinsäuremoleküle, die für Signalpeptide codieren, keine Restriktionsschnittstelle auf, zumindest nicht im Bereich ihrer 3'-Enden. Es ist daher bevorzugt vorgesehen, dass die Schnittstelle für eine Restriktionsendonuklease durch eine stille Punktmutation in der nativen Nukleotidsequenz des Nukleinsäuremoleküls für das Signalpeptid erzeugt ist. Auf diese Weise bleibt die Funktion des Signalpeptides erhalten, und es kommen die oben genannten Vorteile zum Tragen.

Erfindungsgemäß ist weiterhin ein Verfahren zur Herstellung eines Proteins mit Deacetylase-Aktivität vorgesehen, aufweisend die folgenden Schritte:
a) ggf. Klonieren eines Nukleinsäuremoleküls gemäß obiger Definition in einen Expressionsvektor,
b) Transfektion Nukleinsäuremoleküls bzw. des Expressionsvektors in ein Expressionssystem,
c) Inkubation und/oder Fermentation des Expressionssystems;
d) ggf. spezifische Induktion der Proteinexpression;
e) Aufreinigung des exprimierten Proteins mit Deacetylase-Aktivität.

Bei dem exprimierten Protein handelt es sich um eine Chitin-Deacetylase aus Weizenschwarzrost (*Puccinia graminis*).

Unter dem Begriff "klonieren" sollen im folgenden alle Techniken verstanden werden die es erlauben, ein Nukleinsäuremoleküls gemäß obiger Definition in einen Expressionsvektor zu integrieren. Dem Fachmann sind hier eine Vielzahl von Techniken bekannt, deren wesentliche z.B. in dem Lehrbuch "Molekulare Biotechnologie" von Michael Wink (1. Auflage, (September 2004, Wiley-VCH), oder in anderen dem Fachmann bekannten Lehrbüchern und Veröffentlichungen beschrieben sind.

Unter dem Begriff "Expressionsvektor", der optional zu verwenden ist, sollen dem Fachmann an sich bekannte Transportmoleküle zur Übertragung eines Fremd-Nukleinsäuremoleküls in eine Empfängerzelle verstanden werden. Hierzu zählen insbesondere Plasmide, λ-Phagen, Cosmide, Phasmide, P1-Phage, BACs, PACs, und YACs. Weitere geeignete Vektoren sind z.B. in dem Lehrbuch "Molekulare Biotechnologie" von Michael Wink (1. Auflage, (September 2004, Wiley-VCH), oder in anderen dem Fachmann bekannten Lehrbüchern und Veröffentlichungen beschrieben.

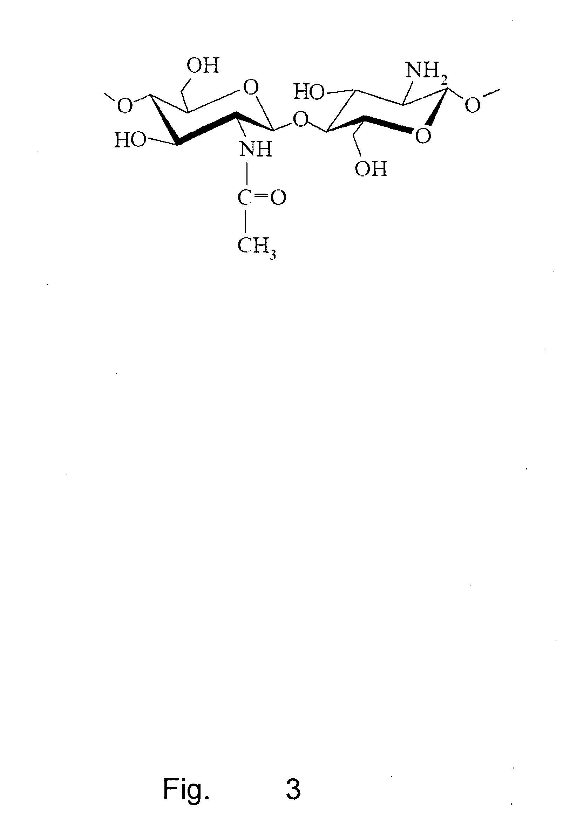

Bei dem optional verwendeten Expressionsvektor handelt es sich bevorzugt um das Plasmid pMel1, das insbesondere in *Schizosaccharomyces pombe* als bevorzugtem Expressionssystem zum Einsatz kommt. Dieses Plasmid ist in Fig. 3 dargestellt.

Unter dem Begriff "Transfektion" soll im folgenden das Einbringen eines Fremd- Nukleinsäuremoleküls in eine Empfängerzelle verstanden werden, insbesondere unter Zuhilfenahme eines Expressionsvektors.

Unter den Begriffen "Inkubation" und/oder Fermentation des Expressionssystems soll imfolgenden das Hältern des Expressionssytems unter Wachstums- und zellteilungsfördernden Bedingungen verstanden werden., Dem Fachmann sind diese Bedingungen (insbesondere pO₂, pH, Temperatur, Medienzusammensetzung, etc.) aus der Fachliteratur bekannt.

Weiterhin ist bevorzugt vorgesehen, dass das exprimierte Protein mit Deacetylase-Aktivität durch Affinitätschromatographie, Gelfiltration und/oder Ionenaustauschchromatographie aufgereinigt wird. Hier kommt insbesondere die Kombination mit einem His-Tag in Frage. Hierbei handelt es sich um ein aus mehreren Histidin-Resten bestehendes Tag, dass an das aufzureinigende Protein angehängt ist. Die hierfür codierende DNA ist in der Regel Bestandteil des Expressionsvektors und wird zusammen mit der cDNA des zu exprimierenden Proteins translatiert.

Grundprinzip dieses Aufreinigungsverfahrens ist die sogenannte "IMAC" (Immobilized Metal Affinity Chromatography). Dabei werden zweifach positiv geladene Kationen wie Cu²⁺, Ni²⁺, Co²⁺ oder Zn^{2◆} über einen chelierenden Liganden an eine Matrix immobilisiert, und interagieren mit den Seitengruppen der Histidin-reste des His-Tag. Weitere, erfindungsgemäß einsetzbare Affinitäts-Tags sind z.B. Maltose-bindendes Protein (MBP) oder Glutathionyl-S-Transferase (GST), aber auch das S-Tag, HAT-Tag, Calmodulin-Binde-Peptid, Chitin-Binde-Peptid und einige Cellulose-Binde-Domänen. Hier kommen ähnliche Aufreinigungsmechanismen zum Einsatz.

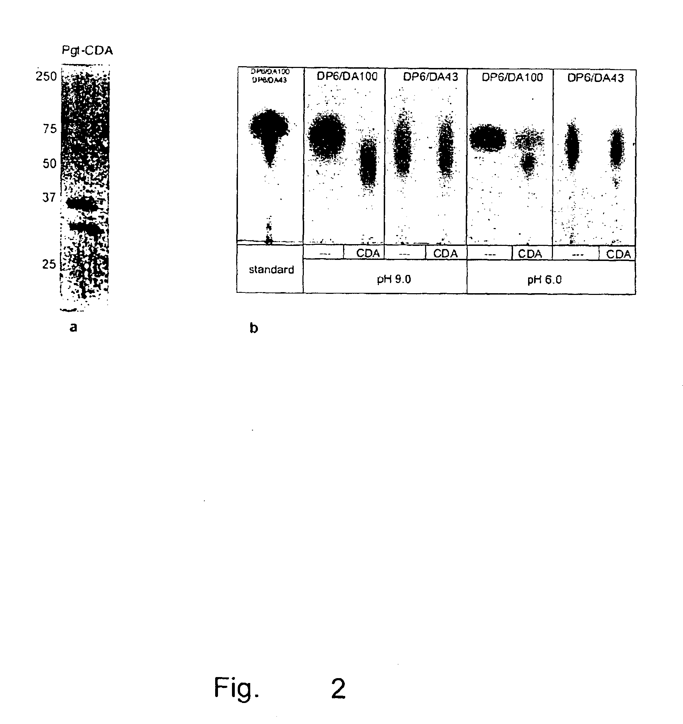

Erfindungsgemäß ist weiterhin ein Expressionsvektor vorgesehen, der das oben beschriebene Nukleinsäuremolekül enthält. Ein solcher Expressionsvektor ist z.B. in Fig. 2 gezeigt.

Ebenso ist erfindungsgemäß eine Wirtszelle, die mit einem solchen Nukleinsäuremolekül in einem solchen Expressionsvektor transformiert wurde, wobei diese Zelle als Expressionssystem fungiert. Bei einer solchen Wirtszelle kann es sich z. B. um eine eukaryontische Wirtszelle handeln. Bevorzugt werden hier Hefezellen verwendet, insbesondere Bierhefe (*Saccharomyces cerevisiae*) und Spalthefe (*Schizosaccharomyces pombe*).

Geeignet sind zwar prinzipiell alle eukaryontischen Expressionssysteme (also Pilze inkl. Hefen, Einzeller, Pflanzen, Insektenzellen und Säugerzellen). In der Regel können funktionelle Proteine jedoch nur in Kombination mit einem im entsprechenden System gut funktionierenden Sekretionssignal exprimiert und sezerniert werden. Es ist daher bevorzugt, den beschriebenen Vektor in anderen Hefen einzusetzen oder das Prinzip zu übertragen. Hierbei ist insbesondere an die folgenden Hefen gedacht: *Pichia pastoris, Hansenula polymorpha, Klyveromyces lactis.*

Erfindungsgemäß ist weiterhin ein Polypeptid bzw. Protein vorgesehen, das durch das oben beschriebene Nukleinsäuremolekül codiert ist oder mit dem oben beschriebenen Verfahren herstellbar ist, wobei dieses Polypeptid bzw. Protein eine Deacetylase-Aktivität aufweist. Bevorzugt weist dieses Polypeptid bzw. Protein eine Aminosäuresequenz auf, die in SEQ ID No 3 dargestellt ist.

Abweichend davon kann das Polypeptid bzw. Protein eine Aminosäuresequenz aufweisen, in welcher ein oder mehrere Aminosäurereste konservativ substituiert sind. In Bezug auf den begriff "konservativ substituiert" wird auf die obigen Ausführungen verwiesen.

Bei diesem Polypeptid bzw. Protein handelt es sich um eine Chitin-Deacetylase aus Weizenschwarzrost (*Puccinia graminis*).

Es ist weiterhin ein Verfahren zur Herstellung von Chitosan aus Chitin vorgesehen, bei welchem N-Acetyl-Glucosaminreste (GlcNAc) in einem GlcNAc-Mono- oder -Oligomer, einem GlcNAc-Polymer, einem Copolymer aus GlcNAc und Glucosamin oder in einem Chitinmolekül mit einer solchen Chitin-Deacetylase deacetyliert werden.

Dabei wird bevorzugt ein pH-Wert zwischen 3 und 12 sowie eine Temperatur zwischen 5 und 80 °C eingestellt. Als Puffer kommen insbesondere Tris, Bicine, Phosphat und Borat in Frage. Die Pufferkonzentration beträgt bevorzugt 10 - 100 mM, ganz besonders bevorzugt 40 - 60 mM. Bevorzugt werden außerdem Kofaktoren verwendet, so z.B. Zn (1mM).

Besonders bevorzugt liegt der pH-Wert im Bereich zwischen 6 und 11, und ganz besonders bevorzugt liegt der pH-Wert zwischen 8 und 10. Besonders bevorzugt liegt die Temperatur im Bereich zwischen 20 und 60 °C, und ganz besonders bevorzugt liegt die Temperatur zwischen 30 und 50 °C.

Die Erfinder haben, um die Verfahrensbedinungen experimentell zu optimieren, Chitohexamere mit einem Acetylierungsgrad von 100% (DP6/DA100) und 42% (DP6/DA42) 27h lang bei 37°C und verschiedenen pH-Werten mit der erfindungsgemäßen Pgt-CDA verdaut. Die Reaktionsprodukte sowie Kontrollansätze ohne Enzym wurden mittels DC aufgetrennt und mittels Fluorescamin-Assay ausgewertet. Dabei wurde bei einem pH-Wert 9,0 die höchste Deacetylierungsaktivität (von 100% auf 64% bzw. von 43% auf 33%) festgestellt, bei pH 6,0 (von 100% auf 84% bzw. von 43% auf 35%) bzw. bei pH 4,0(von 100% auf 99% bzw. von 43% auf 39%) lag die Aktivität deutlich geringer.

Alternativ hierzu ist ein Verfahren zur Herstellung von Chitosan vorgesehen, in welchem N-Acetyl-Glucosamin-Monomere (GlcNAc) mit einer solchen Chitin-Deacetylase deacetyliert werden, um so Glucosamin-Monomere (GlcN) herzustellen, die dann z.B. in einem weiteren Teilschritt mit unbehandelten GlcNAc-Monomeren copolymerisiert werden, um Chitosan zu erhalten.

Ebenso kann jedoch vorgesehen sein, dass die erfindungsgemäße Chitin-Deacetylase zur Acetylierung von Glucosaminresten (GlcN) in einem GlcN-Mono- oder Oligomer, einem GlcN-Polymer, einem Copolymer aus GlcNAc und Glucosamin oder einem Chitosanmolekül verwendet wird.

Durch die Wahl geeigneter (d.h. im Prinzip ungünstiger) Reaktionsbedingungen (Temperatur, pH, Umkehrung des Reaktionsgleichgewichts durch Abfuhr der acetylierten Substrate und Zufuhr deacetylierter Substrate) kann die CDA zu einer Umkehr ihrer Reaktionsrichtung gebracht werden, so wie es in praktisch allen enzymatisch katalysierten Reaktionen der Fall ist. Tut man dies, kann man die CDA zur Reacetylierung insbesondere von Chitosansubstraten nutzen. Die dabei entstehenden Produkte werden im Folgenden "acetylierte Produkte" genannt; es kann sich hier selbstverständlich auch um Chitosane mit unterschiedlichen Acetylierungsmustern und - anteilen handeln, aber insbesondere auch um Chitin. Diese Anwendung ist von hohem allgemeinem Interesse, da sie wiederum zu Produkten mit definiertem Acetylierungsmuster und Acetylierungsanteil definiertem führen kann.

Weiterhin ist erfindungsgemäß ein Chitosan oder ein wie gerade beschrieben acetyliertes Produkt vorgesehen, das mit einem der genannten Verfahren herstellbar ist. Ein solches Chitosan oder acetyliertes Produkt zeichnet sich durch einen genau definierbaren Polymerisations- und Acetylierungsgrad sowie ein genau definierbares Acetylierungsmuster aus.

Schließlich ist erfindungsgemäß die Verwendung eines solchen Chitosans oder acetylierten Produkts für mindestens einen der Zwecke vorgesehen, ausgewählt aus der Gruppe enthaltend:
a) Immobilisierung von Enzymen und Zellen in biotechnologischen Verfahren;
b) Verstärkung und/oder Färbung von menschlichen und/oder tierischem Haar;
c) Haut- und/oder Haarpflege;
d) Schutz und Konservierung von Saatgut und/oder Getreide;
e) Adsorption von Endotoxinen und Nukleinsäuren
f) Papierverarbeitung.

Weitere, erfindungsgemäß vorgesehene Verwendungen umfassen:
a) Polymer-Träger für Katalysatoren;
b) Zwischenprodukt für die Synthese von Feinchemikalien;
c) Zusatz für Molkefutter;
d) Kompostierungsbeschleuniger;
e) Verpackungsmaterial;
f) Klärungsmittel für Säfte;
g) Flockenbildungmittel;
h) diätetische Faser für Nahrungsmittel;
i) Beschichtungsmittel für Nahrungsmittel;
j) Wundauflage;
k) chirurgisches Nahtmaterial;
l) diätetischer Nahrungszusatz;
m) Kontaktlinsen;
n) Lautsprechermembrane;
o) Einbettungsmittel für die Elektronenmikroskopie;
p) oder Fällungsmittel für die Trink- und Abwasserreinigung.
q) Matrizes für das Tissue Engineering
r) Chitosan-Partikel für Drug-, Gene- und Vaccine-Delivery

Viele dieser Verwendungen profitieren von dem genau definierbaren Acetylierungsgrad sowie dem genau definierbaren Acetylierungsmuster der erfindungsgemäß herstellbaren Chitosane, der sich insbesondere in einer definierten Löslichkeit sowie in einer definierten Nettoladung des betreffenden Polymers äußert. Beide Parameter sind entscheidend für die genannten Verwendungszwecke.

### Abbildungen und Beispiele

Die vorliegende Erfindung wird durch die im Folgenden gezeigten und diskutierten Figuren und Beispiele genauer erläutert. Dabei ist zu beachten, dass die Figuren nur beschreibenden Charakter haben und nicht dazu gedacht sind, die Erfindung in irgendeiner Form einzuschränken.

Die Begriffe Chitohexaose und Chitohexose werden in den nachstehenden Beispielen synonym verwendet.

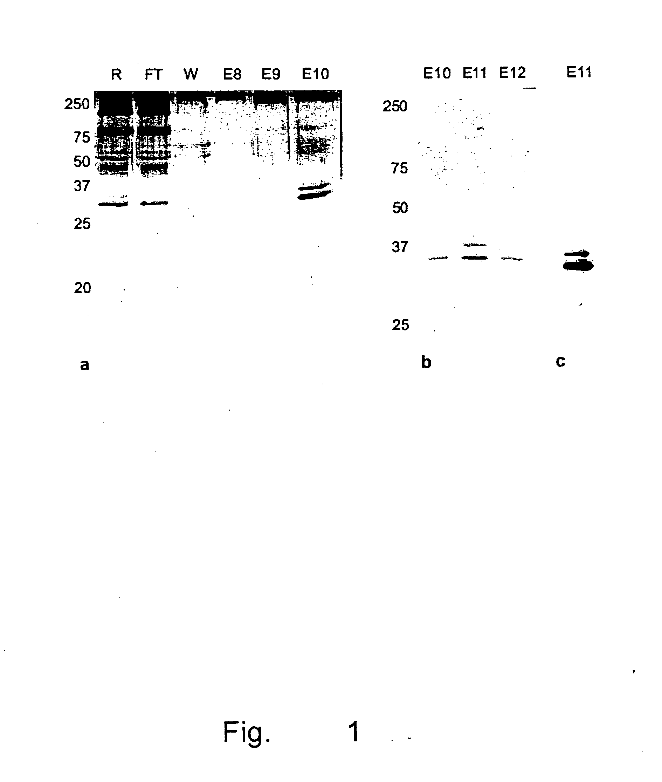

Fig. 1 zeigt ein Screening des Kulturmediums, in welchem mit einem Konstrukt (pMel-CDA) transformierte *Schizosaccharomyces pombe*-Zellen kultiviert wurden. Die Zellen wurden in 10 ml-Kulturen (Falcontubes) kultiviert. Das Kulturmedium wurde 20h nach Induktion untersucht. Die Aufreinigung der erfindungsgemäßen Pgt-CDA aus dem Kulturmedium erfolgte mittels NiNTA-IMAC-Affinitätschromatographie.

Dabei zeigt Fig. 1a) die mit Silber gefärbten PAGE-Gele der wichtigsten Aufreinigungsschritte, Fig. 1b) ein Silbergel der Haupt-Elutionsfraktionen, und Fig. 1c) einen Western-blot der Haupt-Elutionsfraktion (anti-His-tag). Die Gelbahnen sind wie folgt beschickt:
R: Rohfraktion (konz. Kulturmedium)
FT: Durchflussfraktion
W: Waschfraktion
E8 - E12: Elutionsfraktionen

Die Gesamtvolumina der Fraktionen betrugen:
R: 100ml
FT: 100ml
W: 50 ml
E8-12: 1ml

Die Bahnen wurden jeweils mit 15µl Probe beschickt.

Fig. 2 zeigt die Ergebnisse von Aktivitätstest rekombinanter Pgt-CDA gegenüber Chitosan-Substraten. Dabei zeigt Fig. 2a) ein Zymogramm (Substrat: Glykol-Chitin, Inkubation: 37°C in 50 mM BisTris, pH6 über Nacht, Negativfärbung mit Calcofluor nach Depolimerisation von Chitosan mit salpetriger Säure).

Fig. 2b) zeigt die Ergebnisse einer Dünnschichtchromatographie (DC). Dabei wurden Chitohexamere mit einem Acetylierungsgrad von 100% (DP6/DA100) und 42% (DP6/DA42) 27h lang bei 37°C unter zwei verschiedenen Pufferbedingungen (50 mM BisTris, pH 6,0 und 50 mM Borat, pH 9,0) mit rekombinanter Pgt-CDA verdaut. Die Reaktionsprodukte sowie Kontrollansätze ohne Enzym wurden mittels DC aufgetrennt und anschliessend visualisiert.

Die Markierung "---" entspricht einem Ansatz ohne Enzym, die Markierung "CDA" entspricht einem Ansatz mit Enzym.

Fig. 3 zeigt die Strukturformel eines Ausschnitts aus einem Copolymer aus N-Acetyl-Glucosamin (GlcNAc; links) und Glucosamin (GlcN; rechts), die ß-1,4-glycosidisch miteinander verknüpft sind. Dieser Ausschnitt steht idealtypisch für die Struktur von Chitosan.

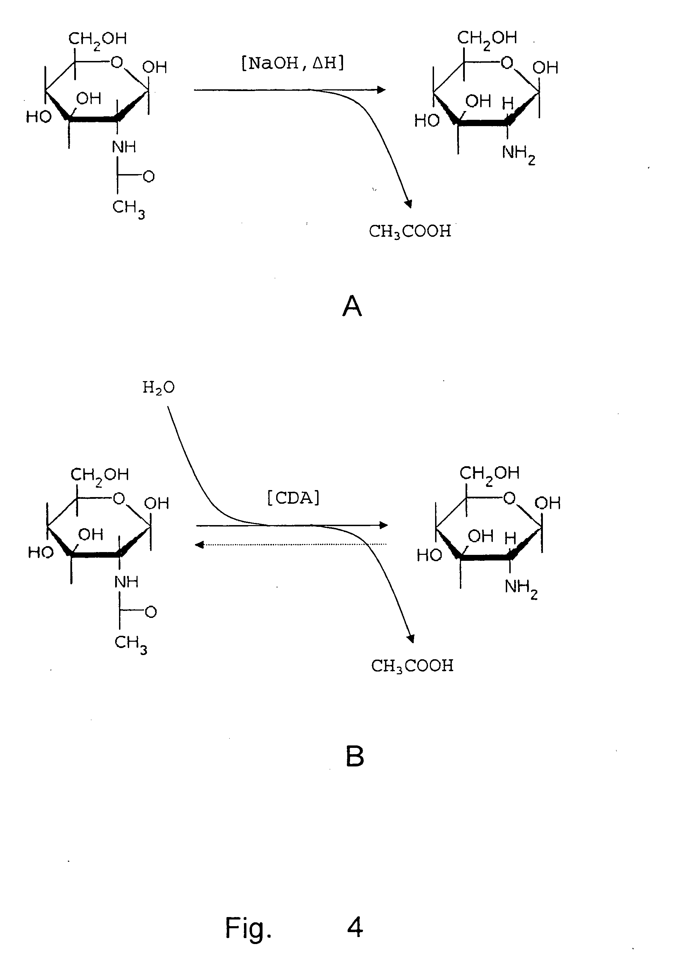

Fig. 4 zeigt das Reaktionsschema der Deacetylierung von N-Acetyl-Glucosamin auf chemischem Wege (Fig.4A) bzw. mit Hilfe der erfindungsgemäßen Chitin-Deacetylase (Fig.4B). Analog gelten die Reaktionsschemata auch für die Deacetylierung von N-Acetyl-Glucosaminresten in einem Chitin- bzw. Chitosanmolekül.

Die Verwendung einer erfindungsgemäßen Chitin-Deacetylase ermöglicht gegenüber der klassischen chemischen Deacetylierung die Herstellung von besser definierten und reproduzierbaren Chitosanen.

Die ebenfalls beschriebene Verwendung der erfindungsgemäßen CDA zur Reacetylierung ist in Fig. 4 durch den unterbrochen dargestellten Reaktionspfeil angegeben.

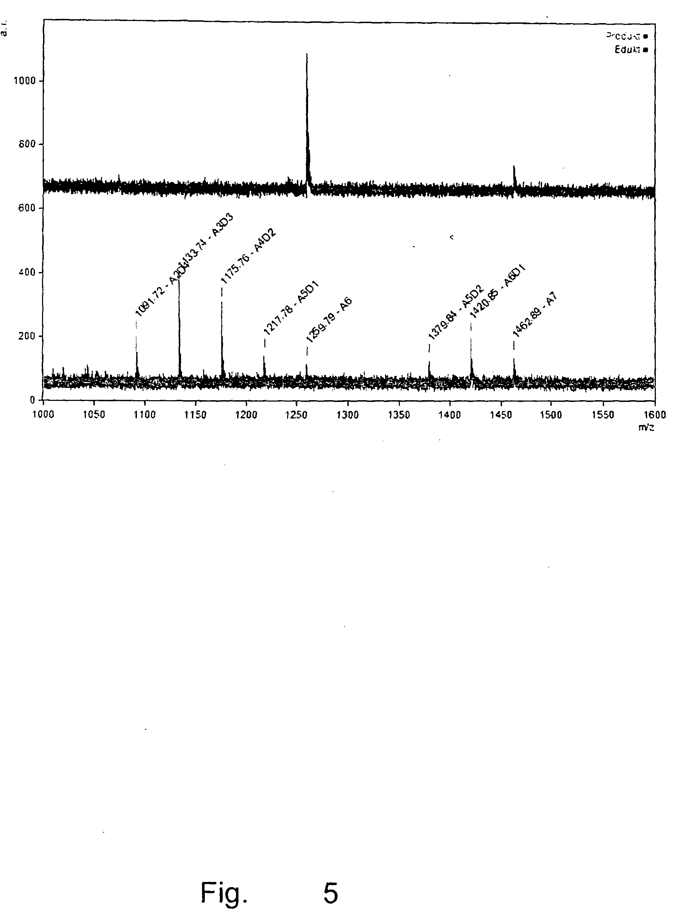

Fig. 5 zeigt die massenspektrometrische Analyse der Reaktionsprodukte. 25 µg Chitohexaose wurden 24h bei 40°C mit 0,5 µg Pgt-CDA in Reaktionspuffer (50 mM BisTris, pH 6,9; 100 mM NaCl; 1 mM ZnCl) inkubiert. Die Reaktionsprodukte, sowie das auf gleiche Weise, aber ohne Enzym inkubierte Edukt wurde mittels Maldi-TOF-MS (Bruker Daltonik Reflex 4) analysiert. Abgebildet sind das Massenspektrum des Edukts (schwarz, oben) sowie das des Produktes (unten, grau).

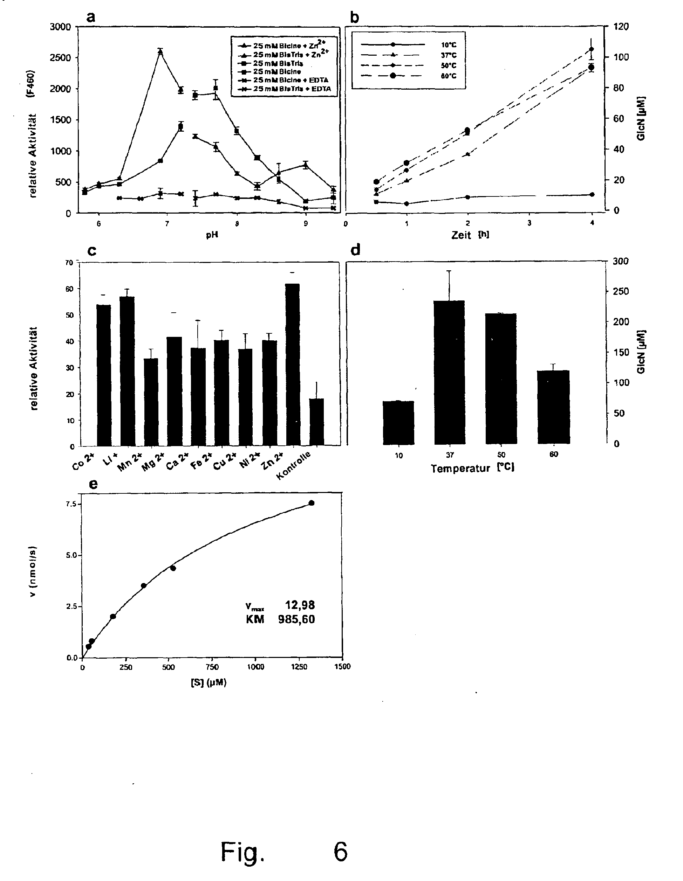

Fig. 6 zeigt die Charakterisierung und Kinetiken von Pgt-CDA. Dabei zeigt Fig. 6a) die Bestimmung des pH-Optimums: 27 nM Pgt-CDA wurde bei unterschiedlichen pH-Werten in verschiedenen Puffern mit 0,88 mM Chitohexose als Substrat bei 37°C für 19 h inkubiert und anschließend die Pgt-CDA-Aktivität bestimmt. Für pH-Werte im Bereich 5,8-7,2 wurden Bis Tris-Puffer, für den Bereich zwischen pH 7,4-9,4 Bicine-Puffer verwendet. Alle Puffer hatten eine Konzentration von 25 mM. Fig. 6b+d) zeigen den Einfluss der Inkubationstemperatur auf die Aktivität: 74 nM Pgt-CDA wurde mit 0,88 mM Chitohexaose in 50 mM BisTris pH 6,9 1 mM ZnCl₂ bei unterschiedlichen Temperaturen im Wasserbad inkubiert. Zu den angegebenen Zeitpunkten wurden Proben genommen, durch Wärmebehandlung inaktiviert und verschlossen bei Raumtemperatur aufbewahrt.

Fig. 6c) zeigt den Einfluss von Metallionen auf die Pgt-CDA Aktivität: 106 nM Pgt-CDA wurde in 50 mM Bicine-Puffer (pH 8,0) bei 37°C für 2 h mit 0,22 mM Chitohexose und verschiedenen Metallionen in Form ihrer Chloridsalze bei einer Konzentration von 1 mM inkubiert. Als Kontrolle wurde 50 mM Bicine-Puffer ohne Metallionen eingesetzt. Fig. 6e)zeigt eine *Michaelis-Menten Kinetik:* 76 nM Pgt-CDA wurde in 50 mM BisTris pH 6,9 1mM ZnCl₂ bei 37 °C für 24 h mit unterschiedlichen Substratkonzentrationen (S) von Chitohexaose inkubiert. KM and vₘₐₓ wurden mittels nichtlinearer Regression mit Hilfe der GraphPad Prism Software ermittelt.

In allen Assays wurde die relative oder absolute Aktivität durch Fluorescamin-Markierung des erhaltenen GlcN und anschließende Fluorometrie bestimmt. Die Werte zeigen den Mittelwert von drei (6a+c) oder zwei (6b,d+e) unabhängigen Reaktionen.

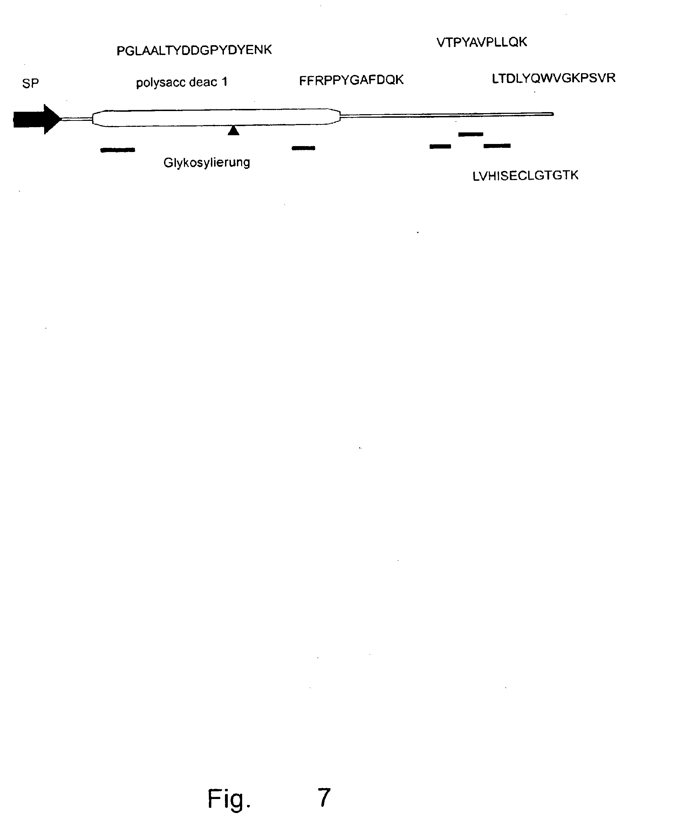

Fig. 7 zeigt die massenspektrometrische Analyse rekombinanter Pgt-CDA. Aufgereinigte, rekombinante Pgt-CDA wurde nach Naumann et al. (2005) analysiert. Gefundene Peptide sowie ihre Position im nativen Protein sind in der Abbildung dargestellt.

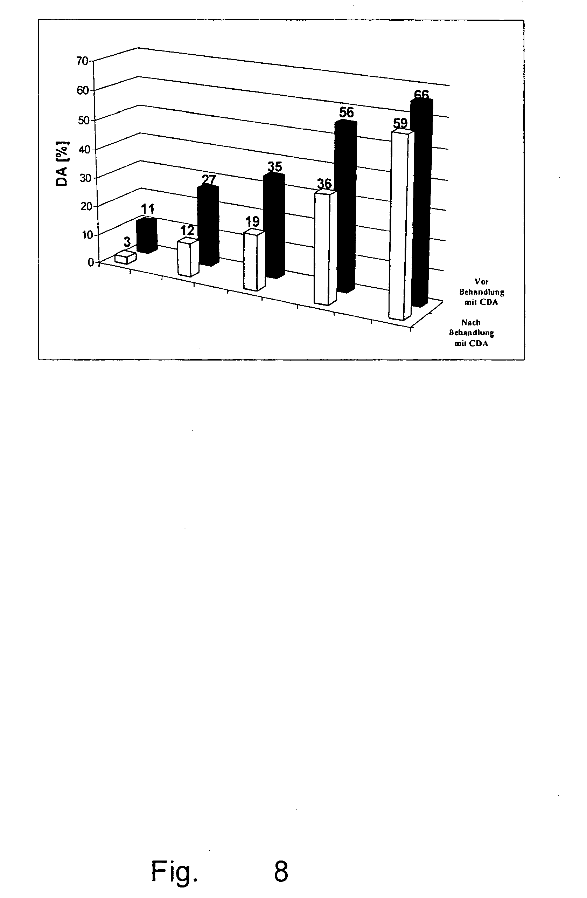

Fig. 8 zeigt die enzymatische Deacytylierung von Chitosan-Substraten. Fünf verschiedene Chitosane wurden durch Pgt-CDA deacetyliert. Die Anfangssubstrate waren (schwarze Balken von Links nach Rechts): DP737/DA11; DP818/DA27; DP713/DA35; DP1442/DA56; DP3726/DA66. Die Substrate wurden 24 h bei 37°C in 50 mM BisTris pH 6,9, 1 mM ZnCl mit 20 nM Pgt-CDA inkubiert. Die Menge an freigesetztem Acetat wurde für jede Reaktion mit Hilfe eines kommerziellen Acetat-Assays (R-Biopharm, Darmstadt) bestimmt und der resultierende Acetylierungsgrad (DA) bestimmt (weiße Balken).

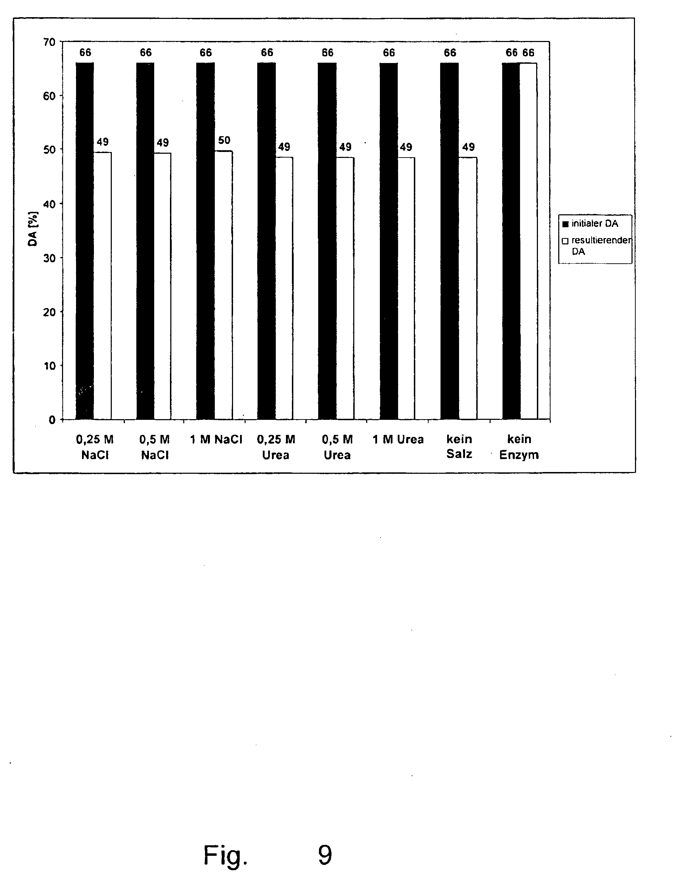

Fig. 9 zeigt die Salztoleranz von Pgt-CDA. Chitosan (DP3726/DA66 - schwarze Balken) wurde mit 80 nM Pgt-CDA in 50 mM BisTris, pH 6,9, 1mM ZnCl und variablen Salzkonzentrationen bei 40°C für 19h inkubiert. Die Menge an freigesetztem Acetat wurde für jede Reaktion mit Hilfe eines kommerziellen Acetat-Assays (R-Biopharm, Darmstadt) bestimmt und der resultierende Acetylierungsgrad (DA) bestimmt .(weiße Balken).

In der Summe demonstrieren die Versuche eine hohe Stabilität des Enzyms in breit variablen abiotischen Bedingungen. Die extreme Salztoleranz in Verbindung mit hoher Thermostabilität und einem breiten pH-Spektrum sollten die Entwicklung eines Ein- bis Zweistufenprozesses zur Deacetylierung kristallinen Chitins ermöglichen.

## Patentansprüche

1. Nukleinsäuremolekül ausgewählt aus der Gruppe bestehend aus:
a) Nukleinsäuremoleküle, die für eine Form des Polypeptids mit der abgeleiteten Aminosäuresequenz gemäß SEQ ID No: 3 codieren, wobei besagtes Polypeptid eine Deacetylase-Aktivität aufweist;
b) Nukleinsäuremoleküle, die die Nukleotidsequenz gemäß SEQ ID No: 1 oder SEQ ID No: 2 aufweisen, und die für eine Form des besagten Polypeptids codieren;
c) Nukleinsäuremoleküle, die für ein Fragment oder ein Derivat eines Polypeptids, das durch ein Nukleinsäuremolekül gemäß a) oder b) codiert wird, codieren, wobei in besagtem Derivat ein oder mehrere Aminosäurereste im Vergleich zu besagtem Polypeptid konservativ substituiert sind, und wobei besagtes Fragment oder Derivat eine Deacetylase-Aktivität hat, und die eine mindestens 95 %ige Sequenzidentität mit einem Nukleinsäuremolekül gemäß a) oder b) aufweisen;
d) oder der komplementäre Strang eines Nukleinsäuremoleküls gemäß a)- c).

2. Nukleinsäuremolekül gemäß Anspruch 1, wobei es sich bei dem codierten Polypeptid mit Deacetylase-Aktivität um eine Chitin-Deacetylase (CDA) handelt.

3. Nukleinsäuremolekül gemäß Anspruch 2, wobei es sich bei dem codierten Polypeptid um eine Chitin-Deacetylase aus Weizenschwarzrost (*Puccinia graminis*) handelt.

4. Nukleinsäuremolekül, **dadurch gekennzeichnet, dass** dieses aus einer sekretorischen Signalsequenz sowie einem Nukleinsäuremolekül gemäß einem der vorherigen Ansprüche besteht.

5. Verfahren zur Herstellung eines Proteins mit Deacetylase-Aktivität, aufweisend die folgenden Schritte:
a) Klonieren eines Nukleinsäuremoleküls gemäß einem der Ansprüche 1 bis 4 in einen Expressionsvektor,
b) Transfektion des Nukleinsäuremoleküls bzw. des Expressionsvektors in ein Expressionssystem,
c) Inkubation und/oder Fermentation des Expressionssystems;
d) ggf. spezifische Induktion der Proteinexpression;
e) Aufreinigung des exprimierten Proteins mit Deacetylase-Aktivität.

6. Verfahren gemäß Anspruch 5, wobei es sich bei dem exprimierten Protein um eine Chitin-Deacetylase aus Weizenschwarzrost (*Puccinia graminis*) handelt.

7. Expressionsvektor, enthaltend das Nukleinsäuremolekül gemäß einem der Ansprüche 1 - 4.

8. Wirtszelle, die mit einem Nukleinsäuremolekül gemäß Anspruch 1 - 4 oder mit dem Expressionsvektor gemäß Anspruch 6 transformiert wurde, wobei diese Zelle als Expressionssystem fungiert.

9. Polypeptid bzw. Protein, dass durch das Nukleinsäuremolekül gemäß Anspruch 1 bis 4 codiert ist, eine Aminosäuresequenz gemäß Anspruch 1 aufweist oder mit dem Verfahren gemäß Anspruch 5 - 6 herstellbar ist, wobei dieses Protein eine Deacetylase-Aktivität aufweist.

10. Polypeptid bzw. Protein gemäß Anspruch 9, wobei es sich bei diesem Protein um eine Chitin-Deacetylase aus Weizenschwarzrost (Puccinia graminis) handelt.

11. Verfahren zur Herstellung von Chitosan aus Chitin, bei welchem N-Acetyl-Glucosaminreste (GlcNAc) in einem GlcNAc-Mono- oder -Oligomer, einem GlcNAc-Polymer, einem Copolymer aus GlcNAc und Glucosamin oder in einem Chitinmolekül mit einem Protein gemäß Anspruch 9 - 10 deacetyliert werden.

12. Verfahren zur Herstellung von Chitosan, in welchem N-Acetyl-Glucosamin-Monomere (GlcNAc) mit einem Protein gemäß Anspruch 9 - 10 deacetyliert werden, um so Glucosamin-Monomere (GlcN) herzustellen, die dann z.B. mit unbehandelten GlcNAc-Monomeren copolymerisiert werden, um Chitosan zu erhalten.

13. Verfahren zur Acetylierung von Glucosaminresten (GlcN) in einem GlcN-Mono- oder Oligomer, einem GlcN-Polymer, einem Copolymer aus GlcNAc und Glucosamin oder einem Chitosanmolekül **dadurch gekennzeichnet, dass** dabei ein Protein gemäß Anspruch 9 - 10 verwendet wird.

14. Chitosan bzw. acetyliertes Produkt, das mit einem der verfahren gemäß einem der Ansprüche 11 - 13 herstellbar ist.

15. Verwendung eines Chitosans bzw. eines acetylierten Produkts gemäß Anspruch 14 für mindestens einen der Zwecke ausgewählt aus der Gruppe enthaltend:
a. Immobilisierung von Enzymen und Zellen in biotechnologischen Verfahren;
b. Verstärkung und/oder Färbung von menschlichen und/oder tierischem Haar;
c. Haut- und/oder Haarpflege;
d. Schutz und Konservierung von Saatgut und/oder Getreide;
e. Adsorption von Endotoxinen und Nukleinsäuren
f. Papierverarbeitung
g. Polymer-Träger für Katalysatoren;
h.Zwischenprodukt für die Synthese von Feinchemikalien;
i. Zusatz für Molkefutter;
j. Komostierungsbeschleuniger;
k. Verpackungsmaterial;
l. Klärungsmittel für säfte;
m. Flockenbildungmittel;
n. diätetische Faser für Nahrungsmittel;
o. Beschichtungsmittel für Nahrungsmittel;
p. Wundauflage;
q. chirurgisches Nahtmaterial;
r. diätetischer Nahrungszusatz;
s. Kontaktlinsen;
t. Lautsprechermembrane;
u. Einbettungsmittel für die Elektronenmikroskopie; oder
v. Fällungsmittel für die Trink- und Abwasserreinigung.
w. Matrizes für das tissue engeneering
x. Chitosan-Nanopartikel für drug-, gene- und vaccine-delivery

## Claims

1. Nucleic acid molecule selected from the group consisting of:
a) Nucleic acid molecules, coding for a type of the polypeptide comprising the deduced amino acid sequence according to SEQ ID No: 3, wherein said polypeptide comprise a deacetylase activity;
b) Nucleic acid molecules, comprising the nucleotide sequence according to SEQ ID No: 1 or SEQ ID No: 2, and coding for a form of said polypeptide;
c) Nucleic acid molecules, coding for a fragment or a derivative of a polypeptide, being coded by a nucleic acid molecule according to a) or b), wherein in said derivative one or more amino acid residues are substituted conservatively in comparison to said polypeptide, and wherein said fragment or derivative comprise a deacetylase activity, and comprise at least 95% sequence identity with a nucleic acid molecule according to a) or b);
d) or a complementary strand of a nucleic acid molecule according to a) - c).

2. Nucleic acid molecule according to claim 1, wherein the coded polypeptide with the deacetylase activity is a chitin-deacetylase (CDA).

3. Nucleic acid molecule according to claim 2, wherein the coded polypeptide is a chitin-deacetylase from stem rusts (Puccinia graminis).

4. Nucleic acid molecule, **characterised in that** said molecule consists of a secretory signal sequence and a nucleic acid molecule according to any one of the previous claims.

5. Process for the production of a protein comprising deacetylase activity, comprising the following steps:
a) cloning of a nucleic acid molecule according to any of the claims 1 - 4 in an expression vector,
b) transfection of the nucleic acid molecule, respectively the expression vector, in an expression system,
c) incubation and/or fermentation of the expression system;
d) if applicable, specific induction of the protein expression;
e) purification of the expressed protein comprising deacetylase activity.

6. Process according to claim 5, wherein the expressed protein is a chitin-deacetylase from stem rusts (Puccinia graminis).

7. Expression vector, comprising the nucleic acid molecule according to any claim 1 - 4.

8. Host cell, wherein the cell was transformed with a nucleic acid molecule according to claims 1 - 4 or with the expression vector according to claim 6, wherein this cell serves as an expression system.

9. Polypeptide respectively protein, that is coded by a nucleic acid molecule according to claims 1 - 4, comprising an amino acid sequence according to claim 1 or being producible according to claims 5 - 6, wherein this protein comprises a deacetylase activity.

10. Polypeptide respectively protein according to claim 9, wherein this protein is a chitin-deacetylase from stem rusts (Puccinia graminis).

11. Process for the production of chitosan from chitin, wherein N-Acetyl-Glucosamin-residues (GlcNAc) in a GlcNAc-mono or -oligomer, a GlcNAc-polymer, a co-polymer from GlcNAc-and glucosamine or in a chitin molecule are deacetylated with a protein according to claims 9 - 10.

12. Process for the production of chitosan, wherein N-Acetyl-Glucosamin-monomers (GlcNAc) are deacetylated with a protein according to claim 9 - 10, in order to produce Glucosamine monomers (GlcN), which are then for instance co-polymerised with untreated GlcNAc - monomers, in order to yield chitosan.

13. Process the acetylation of glucosamine residues (GlcN) in a GlcN-mono- or -oligomer, a GlcN-polymer, a co-polymer from GlcNAc and glucosamine or a chitosan molecule, **characterised in that** a protein according to claims 9 - 10 is used.

14. Chitosan respectively acetylated product, being producible with the process according to the claims 11 - 13.

15. Use of a chitosan respectively an acetylated product according to claim 14 for at least one purpose selected from the group comprising:
a. Immobilising of enzymes or cells in biotechnology processes;
b. Strengthening and/or colouring of human and/or animal hair;
c. Skin - and/or haircare;
d. Protection and preservation of seeds and/or wheat;
e. Absorption of endotoxins and nucleic acids;
f. Paper processing;
g. Polymer scaffold for catalysts;
h. Intermediates for the synthesis of fine chemicals;
i. Supplement to whey feed;
j. Compost accelerator;
k. Packaging material;
l. Clarification agent for juices;
m. Flocculation aid
n. Dietetic fibre for food;
o. Food coating;
p. Wound dressing;
q. Surgical sutures;
r. Dietetic food supplement;
s. Contact lenses;
t. Loudspeaker membrane;
u. Embedding material for electron microscopy;
v. Flocculation aid for drinking and waste water;
w. Matrices for tissue engineering
x. Chitosan nano-particle for drug-, gene- and vaccine delivery

## Revendications

1. Molécule d'acide nucléique sélectionnée dans le groupe consistant en :
a) des molécules d'acide nucléique, qui codent pour une forme du polypeptide ayant la séquence d'acides aminés dérivée selon la SEQ ID N° : 3, ledit polypeptide présentant une activité de désacétylase ;
b) des molécules d'acide nucléique, qui présentent la séquence de nucléotides selon la SEQ ID N° : 1 ou la SEQ ID N° : 2, et qui codent pour une forme dudit polypeptide ;
c) des molécules d'acide nucléique, qui codent pour un fragment ou un dérivé d'un polypeptide, qui est codé par une molécule d'acide nucléique selon a) ou b), dans ledit dérivé un ou plusieurs résidu(s) d'acide aminé étant substitué(s) de façon conservatrice en comparaison audit polypeptide, et ledit fragment ou dérivé ayant une activité de désacétylase, et qui présentent une identité de séquence d'au moins 95 % par rapport à une molécule d'acide nucléique selon a) ou b) ;
d) ou le brin complémentaire d'une molécule d'acide nucléique selon a)-c).

2. Molécule d'acide nucléique selon la revendication 1, s'agissant, en ce qui concerne le polypeptide codé ayant de l'activité de désacétylase, d'une chitine-désacétylase (CDA).

3. Molécule d'acide nucléique selon la revendication 2, s'agissant, en ce qui concerne le polypeptide codé, d'une chitine-désacétylase issue de rouille noire de blé (*Puccinia graminis*).

4. Molécule d'acide nucléique, **caractérisée en ce que** celle-ci est constituée d'une séquence signal de sécrétion ainsi qu'en une molécule d'acide nucléique selon l'une des revendications précédentes.

5. Procédé de préparation d'une protéine ayant de l'activité de désacétylase, comportant les étapes suivantes :
a) clonage d'une molécule d'acide nucléique selon l'une des revendications 1 à 4 dans un vecteur d'expression,
b) transfection de la molécule d'acide nucléique ou du vecteur d'expression, dans un système d'expression,
c) incubation et/ou fermentation du système d'expression ;
d) le cas échéant, induction spécifique de l'expression de protéine ;
e) purification de la protéine exprimée ayant de l'activité de désacétylase.

6. Procédé selon la revendication 5, s'agissant en ce qui concerne la protéine exprimée d'une chitine-désacétylase issue de rouille noire de blé (*Puccinia graminis*).

7. Vecteur d'expression, contenant la molécule d'acide nucléique selon l'une des revendications 1 à 4.

8. Cellule hôte, qui a été transformée avec une molécule d'acide nucléique selon revendication 1 - 4 ou avec le vecteur d'expression selon la revendication 6, ladite cellule agissant comme système d'expression.

9. Polypeptide ou protéine qui est codé(e) par la molécule d'acide nucléique selon revendication 1 à 4, présente une séquence d'acides aminés selon la revendication 1 ou peut être préparé(e) avec le procédé selon la revendication 5 - 6, ladite protéine présentant de l'activité de désacétylase.

10. Polypeptide ou protéine selon la revendication 9, s'agissant en ce qui concerne ladite protéine d'une chitine-désacétylase issue de rouille noire de blé (Puccinia graminis).

11. Procédé de préparation de chitosane à partir de chitine, dans lequel des résidus de N-acétyl-glucosamine (GlcNAc) dans un mono- ou oligomère de GlcNAc, dans un polymère de GlcNAc, dans un copolymère de GlcNAc et de glucosamine ou dans une molécule de chitine, subissent de la désacétylation avec une protéine selon revendication 9 - 10.

12. Procédé de préparation de chitosane, dans lequel des monomères de N-acétyl-glucosamine (GlcNAc) subissent de la désacétylation avec une protéine selon revendication 9 - 10, pour ainsi préparer des monomères de glucosamine (GlcN), qui sont ensuite copolymérisés avec par exemple des monomères de GlcNAc non-traités, pour obtenir du chitosane.

13. Procédé d'acétylation de résidus de glucosamine (GlcN) dans un mono- ou oligomère de GlcN, dans un polymère de GlcN, dans un copolymère de GlcNAc et de glucosamine ou dans une molécule de chitosane, **caractérisé en ce qu'**à cet effet une protéine selon les revendications 9 - 10 est utilisée.

14. Chitosane ou produit acétylé, qui peut être préparé avec un procédé selon l'une des revendications 11 - 13.

15. Utilisation d'un chitosane ou d'un produit acétylé selon la revendication 14 pour au moins un des buts sélectionnés dans le groupe contenant :
a. l'immobilisation d'enzymes et de cellules dans des procédés biotechnologiques ;
b. le renforcement et/ou la coloration de cheveux ou poils humains et/ou animales ;
c. des soins de la peau et/ou des cheveux ;
d. la protection et la conservation de semences et/ou de grains de céréales ;
e. l'adsorption d'endotoxines et d'acides nucléiques
f. le traitement de papier
g. des supports polymères pour catalyseurs ;
h. produit intermédiaire pour la synthèse de produits chimiques fins ;
i. additif pour alimentation animale à base de lactosérum ;
j. des agents accélérateurs de campostage ;
k. matériau d'emballage ;
l. des agents de clarification pour des jus ;
m. des agents de floculation ;
n. des fibres diététiques pour des produits alimentaires ;
o. des agents de revêtement pour des produits alimentaires ;
p. des pansements ;
q. matériel de suture destiné à la chirurgie ;
r. complément alimentaire diététique ;
s. des lentilles de contact ;
t. des membranes de haut-parleur ;
u. des agents d'enrobage pour la microscopie à électrons ; ou
v. des agents de précipitation pour l'épuration de l'eau potable et des eaux usées ;
w. des matrices pour l'ingénierie tissulaire ;
x. des nanoparticules de chitosane pour transport de médicaments, de gènes et de vaccins.


### SEQUENCE LISTING

<110> Westfaelische Wilhelms-universitaet Muenster
<120> pMel-CDA
<130>
<160> 4
<170> PatentIn version 3.3
<210> 1
   <211> 818
   <212> DNA
   <213> Puccinia graminis
<220>
   <221> CDA
   <222> (1)..(818)
   <223> CDNA of Chitosan Deacetylase from Puccinia graminis
<400> 1
   atgaacttcg ttcttcaact cagtgttctg ctgctcacaa tcatctcaca gtcttttgtt      60
   cgatccagtc ccttcacaac aagggcaact gaagttatac ccgatcctgc aaccacctgc     120
   tcccggcccg gccttgcggc acttacttac gatgacggac cttatgatta tgaaaataaa     180
   atctccgatt acctcaatgc ccgtcatatt aaaggcacct tctatgtcaa cggcaataac     240
   tacgattgca tttacgatga ggcgatagtg aagcacttaa agcgcacttt ctcccagggc     300
   cacttgattg gatcacatac atggtcacat gcaaatatca gcagcttatc ggccgcagaa     360
   ttaaaccaac agcttgactt agtcgaagta gcattaatca agattcttgg agtcaagccg     420
   aaattcttcc gacctcctta tggtgcattc gaccagaaga gcttggcgat attgaagaaa     480
   cgggggtaca tcgtggccaa ctggtcattt gactccgaag acgctgtggg agcgacgccc     540
   gagcaatcga tggcatctta taaggaactc tcgaagcagt ttccggcctc tcagatcaca     600
   ctcaaccatg agacttacca aactacggcc gagaaagtga ccccatatgc cgtgcctctg     660
   ttgcaaaagg ccggatataa gctagtgcat atatccgaat gcttgggaac gggcaccaaa     720
   ttgactgatc tctaccaatg ggtcggaaag ccttctgtca gagatgcatc gtggacgtgc     780
   aatgggaccc cagctccaac ggacaactaa gaattccg                             818
<210> 2
   <211> 1205
   <212> DNA
   <213> Puccinia graminis
<220>
   <221> CDA
   <222> (1)..(1205)
   <223> gDNA of Chitosan Deacetylase from Puccinia graminis
<400> 2
   atgaacttcg ttcttcaact cagtgttctg ctgctcacaa tcatctcaca gtcttttgtt     60
   cgatccagtc ccttcacaac aagggcaact gaagttatac ccgatcctgc aaccacctgc    120
   tcccggcccg gccttgcggc acttacttac gatgacggac cttatgatta tgaaaataaa    180
   atctccgatt acctcaatgc ccgtcatatt aaaggcacct tctatgtcaa cggcaataac    240
   tacgattgca tttacgatga ggcgatagtg aagcacttaa agcgcagtaa gaatgttacc    300
   tcttatcaaa aaaatatccc caccattgcc tgcttatcat agattgttgt tggcccaagc    360
   tttctcccag ggccacttga ttggatcaca tagttagtca tacctacccc ctggtttccg    420
   cgagaggtct cttggctcat tgattcttac aacaatcctt cgatgttgcc cggttacaca    480
   atctttgttt agcatggtca catgcaaata tcagcagctt atcggccgca gaattaaacc    540
   aacagcttga cttagtcgaa gtagcattaa tcagttcgtc tcattcaatt tctgtcagac    600
   tttttctccg agtaacatgc ttagtatttt gtgattttgt ttggccatcc agagattctt    660
   ggagtcaagc cgaaattctt ccgacctcct tatggtaagc ttgacgaatt atcaaaccag    720
   atgatgtgtt gttaggattt ttcgctgaag atatgagata attccgccag gtgcattcga    780
   ccagaagagc ttggcgatat tgaagaaacg ggggtacatc gtggccaact ggtcatttga    840
   ctccgaagac gctgtgggag cgacgcccga gcaatcgatg gcatcttata aggaactctc    900
   gaagcagttt ccggcctctc agatcacact caaccatgag acttaccaaa ctacggccga    960
   gaaagtgacc ccatatgccg tgcctctgtt gcaaaaggcc ggatataagc tagtgcatat   1020
   atccgaatgc ttgggaacgg gcaccaaatt gactgatctc taccaatggg tcggaaagcc   1080
   ttctgtcaga gatgtaagtt caagttctgc tcccccaaca gttgaaaaca tcttgaattg   1140
   acttgtggcc ttggattcag gcatcgtgga cgtgcaatgg gaccccagct ccaacggaca   1200
   actaa                                                                1205
<210> 3
   <211> 247
   <212> PRT
   <213> Puccinia graminis
<220>
   <221> CDA
   <222> (1)..(247)
   <223> Chitosan Deacetylase from Puccinia graminis
<400> 3
   Ser Pro Phe Thr Thr Arg Ala Thr Glu Val Ile Pro Asp Pro Ala Thr
   1               5                   10                  15
   Thr Cys Ser Arg Pro Gly Leu Ala Ala Leu Thr Tyr Asp Asp Gly Pro
               20                  25                  30
   Tyr Asp Tyr Glu Asn Lys Ile Ser Asp Tyr Leu Asn Ala Arg His Ile
           35                  40                  45
   Lys Gly Thr Phe Tyr Val Asn Gly Asn Asn Tyr Asp Cys Ile Tyr Asp
       50                  55                  60
   Glu Ala Ile Val Lys His Leu Lys Arg Thr Phe Ser Gln Gly His Leu
   65                  70                  75                  80
   Ile Gly Ser His Thr Trp Ser His Ala Asn Ile Ser Ser Leu Ser Ala
                   85                  90                  95
   Ala Glu Leu Asn Gln Gln Leu Asp Leu Val Glu Val Ala Leu Ile Lys
               100                 105                 110
   Ile Leu Gly Val Lys Pro Lys Phe Phe Arg Pro Pro Tyr Gly Ala Phe
           115                 120                 125
   Asp Gln Lys Ser Leu Ala Ile Leu Lys Lys Arg Gly Tyr Ile Val Ala
       130                 135                 140
   Asn Trp Ser Phe Asp Ser Glu Asp Ala Val Gly Ala Thr Pro Glu Gln
   145                 150                 155                 160
   Ser Met Ala Ser Tyr Lys Glu Leu Ser Lys Gln Phe Pro Ala Ser Gln
                   165                 170                 175
   Ile Thr Leu Asn His Glu Thr Tyr Gln Thr Thr Ala Glu Lys Val Thr
               180                 185                 190
   Pro Tyr Ala Val Pro Leu Leu Gln Lys Ala Gly Tyr Lys Leu Val His
           195                 200                 205
   Ile Ser Glu Cys Leu Gly Thr Gly Thr Lys Leu Thr Asp Leu Tyr Gln
       210                 215                 220
   Trp Val Gly Lys Pro Ser Val Arg Asp Ala Ser Trp Thr Cys Asn Gly
   225                 230                 235                 240
   Thr Pro Ala Pro Thr Asp Asn
                   245
<210> 4
   <211> 818
   <212> DNA
   <213> Schizosaccharomyces pombe and Puccinia graminis
<220>
   <221> pMel-CDA
   <222> (1)..(818)
   <223> construct of a modified Mel1 signal peptide of S.pombe and a chitosan deacetylase of Puccinia graminis cloned at the 3' terminus of Mel1 with help of an artificially introduced restriction site for Nco I
<400> 4
   atgaacttcg ttcttcaact cagtgttctg ctgctcacaa tcatctcaca gtcttttgtt      60
   cgatccagtc ccttcacaac aagggcaact gaagttatac ccgatcctgc aaccacctgc     120
   tcccggcccg gccttgcggc acttacttac gatgacggac cttatgatta tgaaaataaa     180
   atctccgatt acctcaatgc ccgtcatatt aaaggcacct tctatgtcaa cggcaataac     240
   tacgattgca tttacgatga ggcgatagtg aagcacttaa agcgcacttt ctcccagggc     300
   cacttgattg gatcacatac atggtcacat gcaaatatca gcagcttatc ggccgcagaa     360
   ttaaaccaac agcttgactt agtcgaagta gcattaatca agattcttgg agtcaagccg     420
   aaattcttcc gacctcctta tggtgcattc gaccagaaga gcttggcgat attgaagaaa     480
   cgggggtaca tcgtggccaa ctggtcattt gactccgaag acgctgtggg agcgacgccc     540
   gagcaatcga tggcatctta taaggaactc tcgaagcagt ttccggcctc tcagatcaca     600
   ctcaaccatg agacttacca aactacggcc gagaaagtga ccccatatgc cgtgcctctg     660
   ttgcaaaagg ccggatataa gctagtgcat atatccgaat gcttgggaac gggcaccaaa     720
   ttgactgatc tctaccaatg ggtcggaaag ccttctgtca gagatgcatc gtggacgtgc     780
   aatgggaccc cagctccaac ggacaactaa gaattccg                             818